# EUROPEAN PATENT SPECIFICATION

(11) **EP 1 764 056 B1**
(45) Date of publication and mention of the grant of the patent: **09.07.2008**
(21) Application number: 06120803.9
(22) Date of filing: 18.09.2006
(51) Int. Cl.: A61B 17/92, A61B 17/88, F16B 33/02

(54) **Device for removal of fastening means from human tissue**
Vorrichtung zum Entfernen von Befestigungsmitteln aus menschlichem Geweben
Dispositif d'extraction d'éléments de fixation de tissus humains

(30) Priority: 19.09.2005 EP 05108584
(43) Date of publication of application: 21.03.2007
(73) Proprietor: Inion Oy, 33520 Tampere (FI)
(72) Inventor: Happonen, Harri, 33820, TAMPERE (FI); Koho, Petri, 33710, Tampere (FI); Nuutinen, Juha-Pekka, 33210, Tampere (FI); Vuorisalo, Vesa, 33530, Tampere (FI)
(74) Representative: Kaukonen, Juha Veikko

(56) References cited:
- WO-A-03/037192
- US-A- 5 688 285
- US-A- 5 906 616
- US-A- 5 941 885
- US-A- 5 951 554
- US-B1- 6 264 657

## Description

### FIELD OF THE INVENTION

The invention relates to a device for removal of fastening means from human tissue, and more particularly to a device for removal of fastening means from bone tissue, the device comprising a shaft having proximal and distal ends and a longitudinal centre line, the distal end comprising a thread section having a centre line that is coaxial with the longitudinal centre line of the shaft and a left-hand thread that screws itself around the centre line, and a counter surface that is disposed outside the thread and has a surface component direction which intersects one of said centre lines, the counter surface being disposed so as to make contact with the fastening means to be removed when the thread has been rotated into a hole in said fastening means.

### BACKGROUND OF THE INVENTION

Use of biodegradable materials or materials that degrade in the human body is increasing in the manufacture of fastening means used in surgery.

Fastening means, such as screws, pins, anchors, rivets, tacks etc. made of biodegradable materials have the benefit that they degrade within the human body and, therefore, there is no need to remove the fastening means from the human body in another operation. It should be noted that all materials that degrade in the human body are below referred to as 'biodegradable'.

It sometimes happens for one reason or another that a fastening means, for example a screw, breaks during its installation so that a part of the screw remains in a hole into which it was screwed. It is also possible that a head portion of a screw becomes damaged so that the screw cannot be screwed in its intended position in the hole, nor unscrewed out of the hole in order to remove the screw. Furthermore, a fastening means can be broken due to excessive loading or accidents e.g. due to shearing forces, at the fixation interface. Usually, a broken or damaged fastening means needs to be removed from the human body. It is also possible that a fastening means, which is not broken or damaged, is removed from the human body.

Some manufacturers have provided various retraction tools for removing a broken or damaged screw from bone tissue.

US 5,697,935 discloses an apparatus that comprises a hollow boring drill. The drill has an internal cavity and an opening in its distal end to said cavity. The distal end comprises also a cutting surface around the opening. The operator places the cutting surface against bone tissue around the fastening means to be removed, and rotates the drill so as to cut into the bone tissue around the fastening means. The surface of the internal cavity bites and adheres to the external surface of the fastening means. The operator then can move the drill away from the bone tissue and remove the fastening means from the bone tissue.

One of the problems associated with the above apparatus and its use is that a fair quantity of bone tissue needs to be removed during the removal process.

US 6,004,321 discloses an apparatus that is provided for retraction of cannulated screws from bone tissue. The apparatus comprises a reverse, self-tapping helix on a conical surface and a feeler shaft connected to the tip of the conical surface. The feeler shaft guides the apparatus at the entry to the cannula of a fractured screw, and the reverse helix bites to the material of the screw to be removed.

One of the problems associated with the above apparatus is that the reverse helix on a conical surface can expand the biodegradable material, in which case the screw tends to adhere even more to the hole. Consequently, the screw breaks into pieces.

US 6264657 teaches about a threaded extractor, the threads of which can extend in a clock-wise or counter clock-wise manner. The threads are arranged to bite into lip of the spacer while the threads are penetrating into a hole in the spacer.

US 5906616 (The preamble of claim 1 is based on this document) discloses a tool for implantation of a spinal cage. The tool comprises a thread section having reverse threads. The surface of a drive can serve as a counter surface that makes contact with the spinal cage when the thread is rotated into a threaded hole in the cage. Threaded portion of an inner shaft is movably arranged in relation to the drive in the direction of a longitudinal centre line of the inner shaft. The surface of a drive is spring biased relative to the threaded portion.

### BRIEF DESCRIPTION OF THE INVENTION

An object of the present invention is to provide a device so as to alleviate the above disadvantages. The objects of the invention are achieved by a device wherein the thread of the thread section is arranged to bite into the wall of the hole in the fastening means while the thread section is penetrating into said hole.

The invention is based on the idea of using a combination of a left-hand screw and a counter surface in order to remove of a fastening means.

An advantage of the device of the invention is that a broken or damaged fastening means can be removed without damaging or expanding the fixation hole where the fastening means is fastened.

### BRIEF DESCRIPTION OF THE DRAWINGS

In the following, the invention will be described in greater detail by means of preferred embodiments and with reference to the accompanying drawings, in which

Figure 1 is a schematic side view of a device according to the invention,

Figure 2 is a schematic side view of a detail of the device illustrated in Figure 1,

Figure 3 is a schematic and partly cross-sectional side view of a second device according to the invention arranged in a fastening means to be removed from bone tissue,

Figure 4 is a schematic and partly cross-sectional side view of a third device according to the invention arranged in a fastening means to be removed from bone tissue,

Figures 5a to 5e are schematic cross-sectional side views of some other thread designs of devices according to the invention,

Figure 6 is a schematic side view of fourth device according to the invention arranged in a fastening means to be removed from bone tissue,

Figure 7 is a schematic side view of a detail of the device illustrated in Figure 6,

Figure 8 is a second schematic side view of a detail of the device illustrated in Figure 6, and

Figure 9 is a schematic view of a detail of a device according to the invention.

For the sake of clarity, the figures show the invention in a simplified manner. Like reference numbers identify like elements.

### DETAILED DESCRIPTION OF THE INVENTION

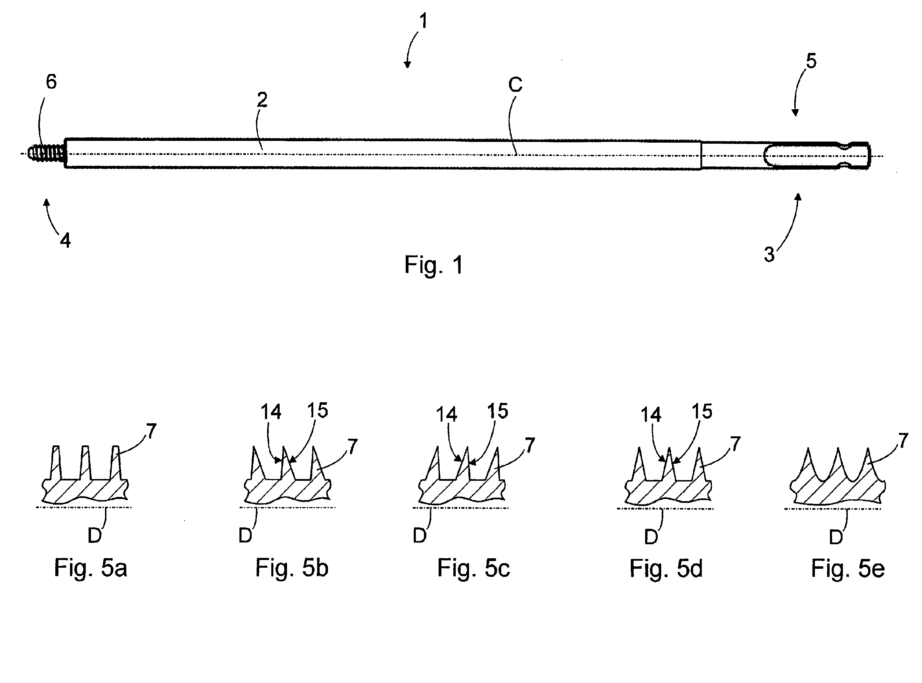

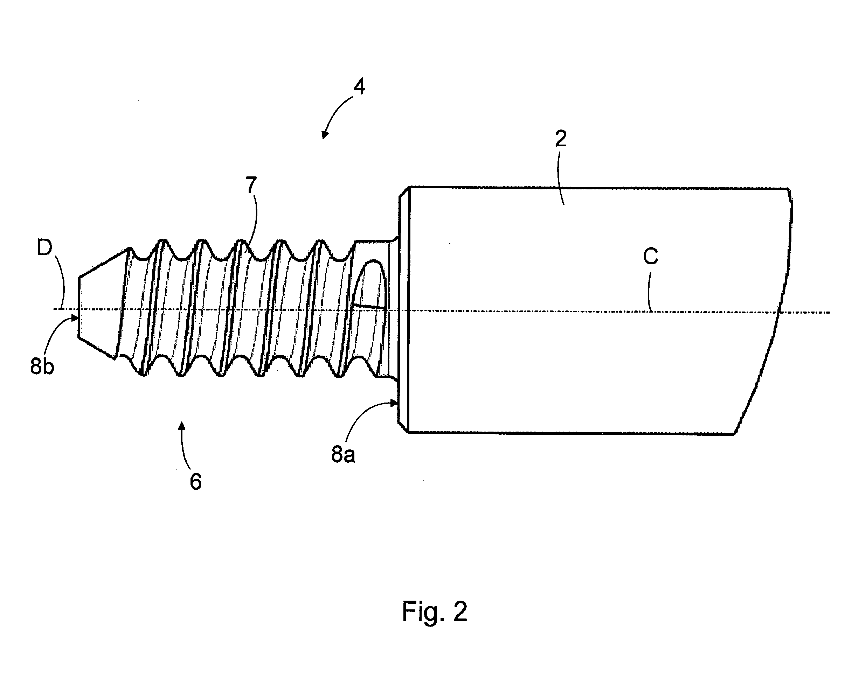

Figure 1 is a schematic side view of a device according to the invention, and Figure 2 is a schematic side view of a detail of the same device.

The device comprises a shaft 2 made e.g. of stainless steel or other similar material that can be used in the manufacture of surgical devices and tools. The shaft 2 has an elongated shape having a proximal end 3 and a distal end 4.

In the proximal end are provided fixing means 5 for fixing the device 1 in a handle or a tool by means of which the device can be rotated around a centre line C of the shaft 2. The tool can be e.g. a drilling machine or a gimlet or some other suitable tool. The fixing means 5 can, of course, be designed in a manner different to that shown in Figure 1. As an alternative, the proximal end 3 can also include a handle that is attached to or integrated in the shaft 2.

It is to be noted that the distance of the proximal end 3 and the distal end 4 can also be considerably shorter than that shown in Figure 1.

The distal end 4 is provided with a thread section 6. As shown in Figure 2, the thread section 6 has a centre line D that is coaxial with the longitudinal centre line C of the shaft 2.

The threaded section 6 is an integral part of the shaft 2 in the device shown in Figure 1. Alternatively, the threaded section 6 can be detachably attached to the shaft 2 by attaching means, e.g. threads.

The thread section 6 includes an external screw thread 7 that screws itself around the centre line D of the thread section 6. The screw thread 7 is a left-hand thread, i.e. opposite to usual threads. Therefore, it must be rotated anti-clockwise in order to screw the thread 7 into a hole.

One screw thread 7 is provided in the thread section 6, extending as a uniform thread from one end to the other end of the thread section 6. The outer diameter of the screw thread 7 is constant, with the exception of a convergent area close to the outmost end of the distal end 4. The height of the screw thread 7 is constant, apart from the convergent area. The height of the screw thread is about 15% of the outer diameter of the thread in the thread section shown in Figure 2. The height of the screw thread 7 may be advantageously selected from a range between 5% and 20%. Such a relatively high thread is preferable in order to prevent the fastening means from expanding in its hole. For the same reason, a relatively thin thread is also preferable.

A pitch of the screw thread 7 is constant throughout the length of the thread section 6.

The distal end 4 is also provided with a first counter surface 8a and a second counter surface 8b. The first counter surface 8a is located between the thread section 6 and the shaft 2. It is a collar-like plane surface perpendicular to the centre line D of the thread section 6. The outer diameter of the first counter surface 8a is substantially larger than that of threaded section 6 and equal to the diameter of the shaft 2. Alternatively, the outer diameter of the first counter surface 8a can be smaller or larger than the diameter of the shaft 2.

The second counter surface 8b is arranged in the outmost end of the distal end 4 of the device 1, at the end of the thread section 6.

Both of the counter surfaces 8a, 8b have a surface component that intersects the centre line of the thread section 6. More accurately, the counter surfaces 8a, 8b are plane surfaces that are arranged perpendicularly to the centre line D of the thread section 6. Alternatively, the first counter surface 8a may resemble a part of a doughnut surface, or a blunt cone surface, for instance, but, nevertheless, the embodiment shown in Figure 2 is usually preferable.

The second counter surface 8b may also resemble a blunt cone or a part of a spherical surface, for instance.

The finish of the counter surfaces 8a, 8b may be roughened up or smoothed, for instance, preferably slightly roughened.

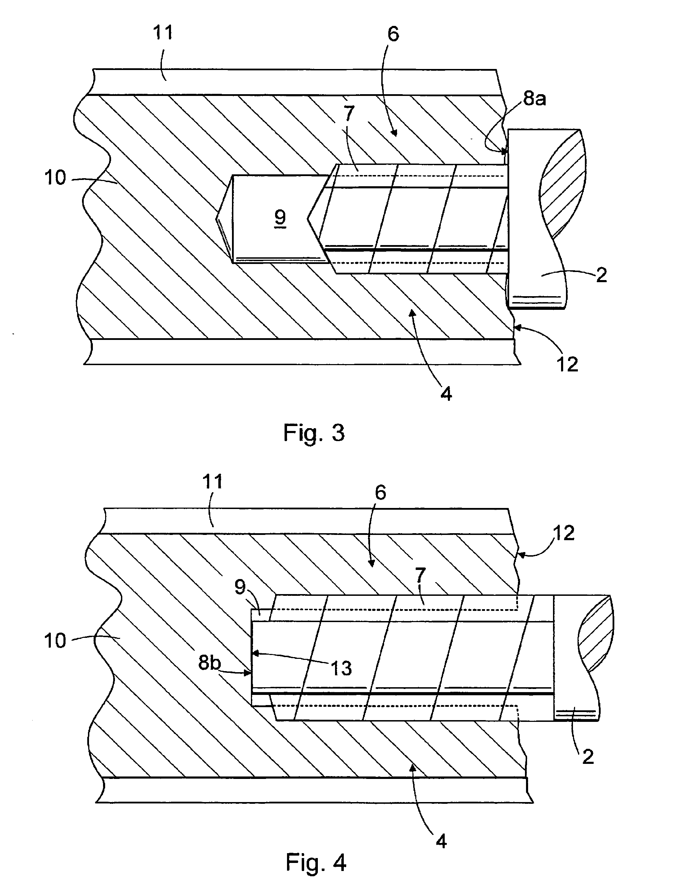

Figure 3 is a schematic and partly cross-sectional side view of a second device according to the invention arranged in a fastening means to be removed from bone tissue. It is to be noted that only the distal end 4 of the device is shown in Figure 3.

The distal end 4 is screwed into a borehole 9 that is made in a fastening means 10 to be removed from bone tissue. The fastening means 10 is a screw made of a biodegradable material. Said bone tissue is not shown in Figure 3, but the screw 10 is fastened to a hole in it by a thread 11. The thread 11 is a right-hand thread, i.e. the screw penetrates into the hole when turned clockwise, and respectively, unscrews out of the hole when turned anti-clockwise.

The screw 10 has fractured along a surface depicted by reference number 12. The part of the screw 10 shown in Figure 3, or at least its thread 11, is jammed in the bone tissue. A jamming force interacts between the screw 10 and the bone tissue and keeps the screw 10 jammed in the bone tissue.

The device of the invention can be used as follows.

The operator has first drilled the borehole 9 in the screw 10. The borehole 9 is preferably made parallel to the longitudinal axis of the screw 10. The borehole is also preferably coaxial with said longitudinal axis. A slight non-paralellism or offset does not cause any harm to the operation.

The borehole 9 can be made with any known drill bit and drilling method and, therefore, this is not discussed in more detail herein. The diameter of the drill bit should, of course, match the dimensions of the thread 7. Especially, the outer diameter of the drill bit is preferably slightly larger than the inner diameter of the thread 7. In this way, the threaded section 6 does not expand radially the screw to be removed.

When the borehole 9 has been finished, the operator takes the drill bit out of the borehole 9. Next, the operator guides the distal end 4 to the open end of the borehole 9, and begins to rotate the thread section 6 into the borehole 9 around centre line D of the thread section. The direction of the rotation is anti-clockwise. The thread 7 of the thread section 6 bites into the wall of the borehole 9 and the thread section 6 penetrates into the borehole 9. In other words, the thread 7 is a self-tapping thread, i.e. the thread 7 has the ability to advance when turned creating its own thread in the borehole 9.

As the operator continues the rotation of the tool, the counter surface 8a is squeezed against a fracture surface 12. This very situation is shown in Figure 3. The operator continues to rotate the device, whereupon the thread 7 of the thread section 6 pulls the counter surface 8a and the fracture surface 12 against each other by a specific pulling force. Due to the pulling force, a frictional force is provided between the counter surface 8a and the fracture surface 12. Another frictional force is also provided between the thread section 6 and the borehole 9. The screw 10 begins to rotate anti-clockwise with the device as a sum of frictional forces between the counter surface 8a and the fracture surface 12, and, on the other hand, between the thread section 6 and the borehole 9 exceeds the above-mentioned jamming force. Thus the screw 10 can be unscrewed out of the borehole in the bone tissue. Ideally, the screw 10 remains in the thread section 6 until the operator removes it therefrom. It is also possible that the fastening means begins to rotate with the device, i.e. unscrew, before the counter surface 8a squeezes against the fastening means.

It is to be noted that the device is not only suitable for removal of broken fastening means but it is also suitable for removal of fastening means whose head is damaged as well as fastening means not damaged at all but which still need to be removed from a human or animal body. The device is suitable for removal of any fastening means made of a biodegradable material or even fastening means made of any biostabile polymer material into which the threaded section can be attached that needs to be removed from a human or animal body.

The device is also suitable for removal of cannulated fastening means. In this case, it is not always necessary to drill a borehole in the fastening means prior to insertion of the thread section 6. Instead, the cannula of the fastening means can be exploited as a hole where the thread section 6 is inserted in.

It is to be noted that the term "fastening means" refers to screws, pins, pegs, anchors, rivets, tacks etc. For example, if a need exists for removal of a pin that is not fixed to tissue with any screw thread, the pin may still be screwed out of its attachment hole. Alternatively, as the thread section 6 is attached firmly to the pin, the pin is simply pulled out of the hole with the help of the device.

It is also to be noted that the first counter surface 8a shown in Figure 2 functions as disclosed above if it is the first counter surface of the two counter surfaces 8a, 8b that squeezes against the fastening means to be removed.

An embodiment of the device of the invention has a counter surface 8a arranged between a thread section 6 and a shaft 2, i.e. similarly to the embodiment shown in Figure 3. In addition a drill bit is provided that is disposed at the outmost end of the distal end. The drill bit has a left-hand cutting edge. Such a device first cuts a borehole in a fastening means to be removed and then bites to said borehole with the thread section, all being provided within the same device and with the same rotation movement.

Figure 4 is a schematic and partly cross-sectional side view of a third device according to the invention arranged in a fastening means to be removed from bone tissue. The device comprises a shaft 2, shown only in part, and a distal end 4 having a threaded section 6 with a left-hand screw thread 7. The distal end 4 also comprises a counter surface 8b fitted at the outmost end of the distal end 4.

Basically, the device shown in Figure 4 functions in the same way as the device shown in Figure 3. The main difference is that because the counter surface 8b is situated at the outmost end of the distal end 4, it squeezes against a bottom 13 of the borehole 9. Accordingly, the thread 7 of the thread section 6 pushes the counter surface 8b against the bottom 13 of the borehole 9, and as soon as a sum of frictional forces between the counter surface 8b and the bottom 13 of the borehole, and, on the other hand, between the thread section 6 and the borehole 9 exceeds the jamming force of the fastening means 10, which is a screw in Figure 4, the fastening means 10 begins to screw itself out of the tissue.

It is to be noted that the second counter surface 8b shown in Figure 2 functions as discussed above if it is the first counter surface of the two counter surfaces 8a, 8b that squeezes against the fastening means to be removed.

Figures 5a to 5e are schematic cross-sectional side views of some other thread designs of devices according to the invention. The thread 7 of the thread section 6 can be realized in various ways. Figures 5a to 5e show some examples of this. In Figure 5a the thread 7 is a trapezoid thread.

In figure 5b, the angle between a leading face 14 of the thread 7 and a plane perpendicular to the centre line D of the threaded section is smaller than the angle of a back face 15 of the thread 7. Contrary to this, the angle between the leading face 14 of the thread 7 and a plane perpendicular to the centre line D of the thread section is larger than the angle of the back face 15 of the thread 7 in Figure 5c. In Figure 5d, the leading face 14 and the back face 15 are arranged into a very same angle with that of a plane perpendicular to the centre line D. In Figure 5e, the profile of the thread 7 has a sharp outer edge and curved leading and back faces.

The thread section 6 may also have two or more screw threads 7, in which case it is a multi-end threaded section. The screw thread 7 can be discontinuous such that at one or more points, it is completely or partially cut. In addition, the profile, pitch, height and thickness of the screw thread 7 may vary throughout the length of the threaded section.

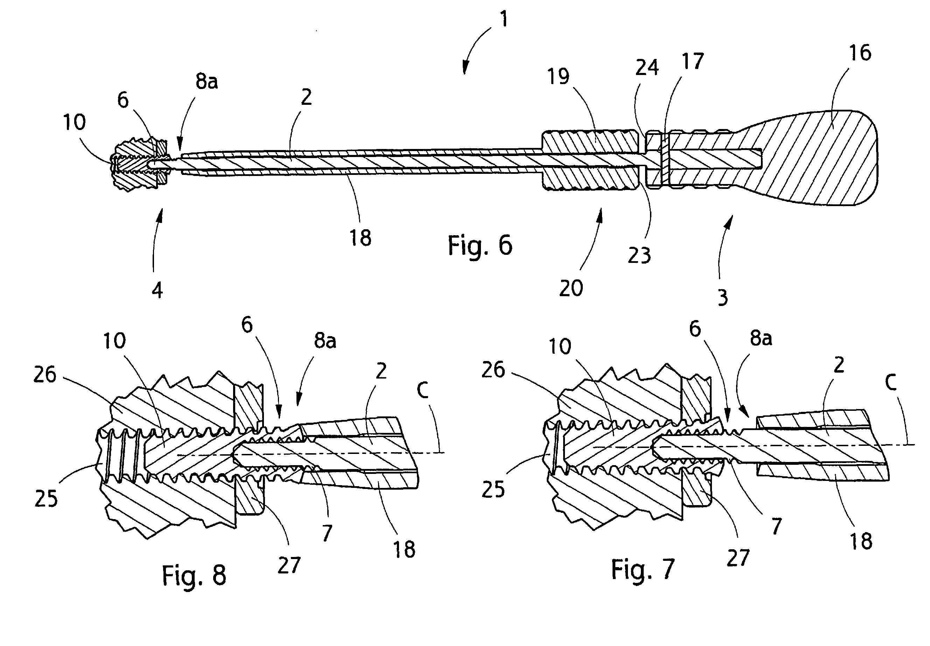

Figure 6 is a schematic cross-sectional side view of a fourth device according to the invention, arranged in a fastening means to be removed from bone tissue, Figures 7 and 8 are schematic views of details of the device illustrated in Figure 6.

The device 1 has a shaft 2 having a proximal end 3 and a distal end 4. The proximal end 3 is provided with a handle 16.

The handle 16 and the shaft 2 have been made separately and attached to each other by attaching means 17. The handle 16 can also be integrated into the shaft 2. A further alternative is to implement the proximal end 3 in a way similar to that in Figure 1.

The distal end 4 is provided with a thread section 6 having an external screw thread 7. The screw thread 7 is a left-hand thread.

The device 1 comprises a sleeve 18 concentrically arranged on the shaft 2. The sleeve 18 can be made of any suitable metal, plastic or combination thereof. The wall of the sleeve 18 can be solid or it can comprise a varying number of punctures or openings.

The proximal end of the sleeve 18 is provided with a grip 19 to facilitate the use of the device 1.

The distal end of the sleeve 18 is provided with a first counter surface 8a. In this embodiment the first counter surface 8a has the shape of an inward bevelled or concave surface which has a surface component that intersects with the centre line of the thread section 6.

A second thread section 20 is arranged on the shaft 2, and its counter thread on the interior surface of the proximal end of the hollow sleeve 18. The second thread section 20 has a left-hand thread.

Thanks to the second thread section 20 and its counter thread, the sleeve 18 and the counter surface 8a thereof can be moved on the shaft 2 in the direction of the longitudinal centre line C by rotating the sleeve with respect to the shaft 2, or rotating the shaft 2 with respect to the sleeve 18.

It is to be noted that the means for moving the first counter surface 8a in relation to the shaft 2 and the thread section 6 thereof can also be implemented in other ways. For example, instead of a counter thread, the sleeve 18 may be provided with one or more protrusions or pins. The protrusion or pin is mounted to slide in the second thread section 20 under the guidance of its threads.

In another embodiment of the tool 1, the sleeve 18 is moved in relation to the shaft 2 with a linear motion, without any substantial rotational movement, i.e. no second thread section or any other means for forcing the sleeve to rotate in relation to the shaft 2 is provided. In this embodiment, the means for moving the first counter surface 8a in relation to the shaft 2 and thread section 6 comprise locking means enabling the operator to lock the first counter surface 8a into a suitable position, preferably in a steplessly way, in relation to the thread section 6. The locking means can comprise, for instance, a lever in the sleeve 18 that can be forced against the shaft 2. The tool 1 can be provided with guiding means to prevent the sleeve 18 from rotating in relation to the shaft 2 during the linear motion. The guiding means can be implemented e.g. by a pin in the shaft 2 and a longitudinal slit or groove in the sleeve 18, the pin being arranged to move in the slit or groove.

In still another embodiment, an elastic member is provided between the shaft 2 and the sleeve 18, for instance between ends 23 and 24. The elastic member can be, for example, a spring or a block of elastic material, such as rubber. The elastic member tends to move the first counter surface 8a towards the distal end of the shaft 2. The tool 1 can comprise locking means to enable the operator to lock and keep the elastic element in a compressed state. The operator can release the elastic member, i.e. allow the first counter surface 8a to pursue towards the distal end of the shaft 2, in any stage of the removal operation. It is to be noted, however, that the locking means are not among the necessary features of the tool.

In still another embodiment, the first counter surface 8a is detachably arranged in the tool 1. The operator can attach or detach the counter surface 8a in the shaft 2 in any stage of the removal operation. The device shown in Figures 6 to 8 can be used as follows. The operator drills, if necessary a borehole in the fixation means to be removed in a way similar to that discussed earlier in this description. It may not be necessary to make the borehole if the fastening means is a cannulated one. The fixation means is a screw 10 for which a threaded fixation hole 25 has been made in a tissue 26. Typically the tissue 26 is bone tissue or cartilage tissue, or the fixation hole has been made partly in bone tissue and partly in cartilage tissue.

The screw 10 is provided for attaching a fixation plate 27 to the tissue 26. For one reason or another, the screw 10 could not be screwed into the fixation hole 25 deep enough, but has become stuck or jammed the position shown in Figure 7. Therefore, the screw 10 needs to be removed from the fixation hole 25.

The hollow sleeve 18 is rotated towards the handle 16 so that the proximal end 23 of the grip 19 is locked by friction against the distal end 24 of the handle 16.

The thread section 6 is guided into the borehole by rotating the device 1 anti-clockwise. The thread section 6 penetrates deeper in the borehole while the self-tapping thread 7 cuts its way into the wall of the borehole. The operator can rotate the device 1 by gripping either the handle 16 or the grip 19.

The rotation of the shaft 2 is stopped as the thread section 6 has penetrated deep enough into the borehole, or the thread section 6 has reached the bottom of the borehole.

Next, the frictional locking between the shaft 2 and the sleeve 18 is released by turning the sleeve 18 anti-clockwise in relation to the shaft 2.

If the operator has rotated the device 1 by the grip 19, it is also possible that the frictional force between the thread section 6 and the borehole in the screw 10 exceeds the frictional force between the proximal end 23 of the grip 19 and the distal end 24 of the handle 16, in which case the locking of the sleeve 18 in the shaft 2 becomes released.

The rotation of the sleeve 18 is continued until the second counter surface 8a comes into contact with the screw 10 to be removed. The screw 10 begins to rotate anti-clockwise together with the device 1 as the frictional forces between the device 1 and the screw 10 exceed the jamming force affecting between the screw 10 and the tissue 26 and/or the plate 27. The operator can rotate the tool 1 by the grip 19, by the handle 16, or he or she can rotate both the grip 19 and the handle 16 at the same time. In Figure 8, the screw 10 has already been partly screwed out of the borehole 25.

It is to be noted that also other ways exist to utilize the device shown in Figures 6 to 8. For example, it is not necessary to lock the proximal end 23 of the grip 19 against the distal end 24 of the handle 16. It will suffice to move the sleeve 18 towards the handle 16 to the extent that a sufficient length of the thread section 6 is exposed so as to enable the operator to begin to screw the thread 7 into the borehole.

Alternatively, the second thread section 20 can be a right-hand thread. This kind of tool can be used such that the operator rotates the shaft 2 and supports the rotating shaft 2 by the sleeve 18, which he or she does not allow to rotate together with the shaft 2. The right-hand thread forces the sleeve 18 towards the fixation means to be removed. When the fixation means begins to rotate in the borehole, the operator can leave the sleeve 18 to rotate with the shaft 2.

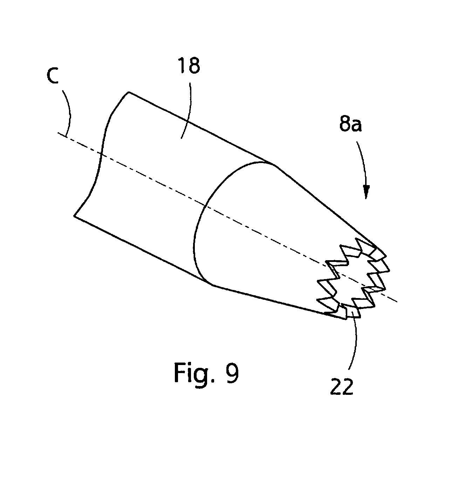

A variant of the counter surface 8a of the device illustrated in Figure 6 is shown in Figure 9. The counter surface 8a situated at the distal end of the sleeve 18 comprises a plurality of protrusions 22 extending in the direction of the longitudinal centre line C. The protrusions 22 which resemble look like sharp teeth, bite into the proximal head of the fastening means to be removed when the sleeve 18 is rotated towards the distal end of the shaft 2. It is to be noted that only a part of the sleeve 2 is shown in Figure 9, and also that no shaft 2 is shown in Figure 9 at all.

The shape and number of protrusions 22 can, of course, be different from those of the counter surface 8a shown in Figure 9.

The distal end of the sleeve 18 has a tapered shape, but this is not necessary. Nevertheless, a tapered shape is sometimes advantageous because it fits better into small boreholes than a non-tapered one does.

It will be obvious to a person skilled in the art that as technology advances, the inventive concept can be implemented in various ways. The invention and its embodiments are not limited to the examples described above but may vary within the scope of the claims.

In an embodiment of the tool 1, the screw thread 7 is a right-hand thread, i.e. as in conventional threads. This kind of tool can be utilized for removing of fastening means with no threads at all, e.g. pins, pegs, rivets, tacks etc., and fastening means which attach to tissue by a left-hand thread. 2)

## Claims

1. A device for removal of fastening means from human tissue, the device comprising
a shaft (2) having proximal and distal ends (3, 4) and a longitudinal centre line (C),
the distal end (4) comprising a thread section (6) having a centre line (D) that is coaxial with the longitudinal centre line (C) of the shaft (2) and
a left-hand thread (7) that screws itself around the centre line (D), and
a counter surface (8a, 8b) that is disposed outside the thread (7) and has a surface component direction which intersects one of said centre lines (C, D),
the counter surface (8a, 8b) being disposed so as to make contact with the fastening means to be removed when the thread (7) has been rotated into a hole in said fastening means,
the thread (7) of the thread section (6) comprising self-tapping thread(s),
the counter surface (8a, 8b) being movably arranged in relation to the thread section (6) in the direction of the longitudinal centre line (C),
the device comprising means for moving the counter surface (8a, 8b) in relation to the thread section (6), said means for moving the counter surface (8a, 8b) in relation to the thread section (6) comprising a sleeve (18) movably disposed on the shaft (2), **characterized in that**
said means for moving the counter surface (8a, 8b) in relation to the thread section (6) comprises:
a second thread section (20) fitted to the shaft (2) or to the sleeve (18) and its counter means disposed on the sleeve (18) or on the shaft (2), respectively, wherein
movement of the counter surface (8a) in relation to the thread section (6) takes place by rotating the shaft (2) and the sleeve (18), in relation to each other, around a rotation axis parallel to the direction of the longitudinal centre line (C).

2. A device as claimed in claim 1, **characterized in that** the thread (7) is fitted between the counter surface (8a8b) and an outmost end of the distal end (4) of the device.

3. A device as claimed in claim 1, **characterized in that** it comprises two counter surfaces: a first counter surface (8a) arranged such that the thread (7) is located between it and the outmost end of the distal end (4), and a second counter surface (8b) fitted at the outmost end of the distal end (4).

4. A device as claimed in any one of claims 1 to 3, **characterized in that** an outer diameter of the thread (7) is invariable substantially throughout its length.

5. A device as claimed in any one of the preceding claims, **characterized in that** a pitch of the thread (7) is constant throughout its length.

6. A device as claimed in claim 1, **characterized in that** the thread (7) is fitted between the counter surface (8a) and the outmost end of the distal end (4) of the device, and a drill bit is disposed at the outmost end of the distal end (4), the drill bit having a left-hand cutting edge.

7. A device as claimed in any one of the preceding claims, **characterized in that** the counter surface (8a, 8b) is provided with protrusions extending in the direction or the longitudinal centre line (C).

8. A device as claimed in any one of the preceding claims, **characterized in that** the second thread section (20) has a left-hand thread.

9. A device as claimed in any one of the preceding claims, **characterized in that** the second thread section (20) has a right-hand thread.

## Patentansprüche

1. Vorrichtung zum Entfernen von Befestigungsmitteln aus menschlichem Gewebe, wobei die Vorrichtung umfasst:
einen Schaft (2), der ein proximales und ein distales Ende (3, 4) und eine Längsmittelachse (C) aufweist,
wobei das distale Ende (4) einen Gewindeabschnitt (6) umfasst, der eine Mittelachse (D) aufweist, die gleichachsig mit der Längsmittelachse (C) des Schaftes (2) ist, und
ein Linksgewinde (7), das sich um die Mittelachse (D) windet, und
eine Gegenfläche (8a, 8b), die außerhalb des Gewindes (7) angeordnet ist und die eine Oberflächenkomponentenrichtung aufweist, die eine der Mittelachsen (C, D) kreuzt,
wobei die Gegenfläche (8a, 8b) so angeordnet ist, dass sie Kontakt mit dem Befestigungsmittel herstellt, das entfernt werden soll, wenn das Gewinde (7) in ein Loch in dem Befestigungsmittel gedreht worden ist,
wobei das Gewinde (7) des Gewindeabschnitts (6) (ein) selbstschneidende(s) Gewinde umfasst,
wobei die Gegenfläche (8a, 8b) in Bezug auf den Gewindeabschnitt (6) in die Richtung der Längsmittelache (C) beweglich angeordnet ist,
wobei die Vorrichtung eine Einrichtung zum Bewegen der Gegenfläche (8a, 8b) in Bezug auf den Gewindeabschnitt (6) umfasst, wobei die Einrichtung zum Bewegen der Gegenfläche (8a, 8b) in Bezug auf den Gewindeabschnitt (6) eine Hülse (18) umfasst, die beweglich auf dem Schaft (2) angeordnet ist,
**dadurch gekennzeichnet, dass**
die Einrichtung zum Bewegen der Gegenfläche (8a, 8b) in Bezug auf den Gewindeabschnitt (6) umfasst:
einen zweiten Gewindeabschnitt (20), der an dem Schaft (2) oder der Hülse (18) angebracht ist, und seine Gegeneinrichtung, die entsprechend an der Hülse (18) oder an dem Schaft (2) angeordnet ist, wobei
die Bewegung der Gegenfläche (8a) in Bezug auf den Gewindeabschnitt (6) durch Drehen des Schaftes (2) und der Hülse (18) im Verhältnis zueinander um eine Drehachse parallel zu der Richtung der Längsmittelachse (C) erfolgt.

2. Vorrichtung nach Anspruch 1, **dadurch gekennzeichnet, dass**
das Gewinde (7) zwischen der Gegenfläche (8a, 8b) und einem äußersten Ende des distalen Endes (4) der Vorrichtung angebracht ist.

3. Vorrichtung nach Anspruch 1, **dadurch gekennzeichnet, dass**
sie zwei Gegenflächen umfasst: eine erste Gegenfläche (8a), die so angeordnet ist, dass das Gewinde (7) sich zwischen ihr und dem äußersten Ende des distalen Endes (4) befindet, und eine zweite Gegenfläche (8b), die an dem äußersten Ende des distalen Endes (4) angebracht ist.

4. Vorrichtung nach einem der Ansprüche 1 bis 3, **dadurch gekennzeichnet, dass**
ein Außendurchmesser des Gewindes (7) im Wesentlichen über seine gesamte Länge gleichbleibend ist.

5. Vorrichtung nach einem der vorhergehenden Ansprüche, **dadurch gekennzeichnet, dass**
eine Steigung des Gewindes (7) im Wesentlichen über seine gesamte Länge gleichbleibend ist.

6. Vorrichtung nach Anspruch 1, **dadurch gekennzeichnet, dass**
das Gewinde (7) zwischen der Gegenfläche (8a) und dem äußersten Ende des distalen Endes (4) der Vorrichtung angebracht ist und eine Bohrspitze an dem äu-ßersten Ende des distalen Endes (4) angeordnet ist, wobei die Bohrspitze eine Linksschneide aufweist.

7. Vorrichtung nach einem der vorhergehenden Ansprüche, **dadurch gekennzeichnet, dass**
die Gegenfläche (8a, 8b) mit Vorsprüngen ausgestattet ist, die sich in die Richtung der Längsmittelachse (C) erstrecken.

8. Vorrichtung nach einem der vorhergehenden Ansprüche, **dadurch gekennzeichnet, dass**
der zweite Gewindeabschnitt (20) ein Linksgewinde aufweist.

9. Vorrichtung nach einem der vorhergehenden Ansprüche, **dadurch gekennzeichnet, dass**
der zweite Gewindeabschnitt (20) ein Rechtsgewinde aufweist.

## Revendications

1. Dispositif d'extraction d'éléments de fixation de tissus humains, le dispositif comprenant :
une tige (2) présentant des extrémités proximale et distale (3, 4) et une ligne centrale longitudinale (C),
l'extrémité distale (4) comprenant une section filetée (6) présentant une ligne centrale (D) qui est coaxiale par rapport à la ligne centrale longitudinale (C) de la tige (2) et
un filetage à gauche (7) qui se visse autour de la ligne centrale (D), et
une contre-surface (8a, 8b) qui est disposée en dehors du filetage (7) et qui présente une direction de surface qui coupe une desdites lignes centrales (C, D),
la contre-surface (8a, 8b) étant disposée de façon à entrer en contact avec le moyen de fixation à extraire lorsque le filetage (7) a été introduit par rotation dans un trou dans ledit moyen de fixation,
le filetage (7) de la section filetée (6) comprenant un ou plusieurs filet(s) autotaraudeur (s),
la contre-surface (8a, 8b) étant disposée de façon amovible par rapport à la section filetée (6) dans la direction de la ligne centrale longitudinale (C),
le dispositif comprenant un moyen permettant de déplacer la contre-surface (8a, 8b) par rapport à la section filetée (6), ledit moyen permettant de déplacer la contre-surface (8a, 8b) par rapport à la section filetée (6) comprenant un manchon (18) disposé de façon mobile sur la tige (2), **caractérisé en ce que**
ledit moyen permettant de déplacer la contre-surface (8a, 8b) par rapport à la section filetée (6) comprend :
une seconde section filetée (20) adaptée sur la tige (2) ou sur le manchon (18) et son contre-moyen disposé sur le manchon (18) ou sur la tige (2), respectivement, dans lequel
le déplacement de la contre-surface (8a) par rapport à la section filetée (6) se produit en faisant tourner la tige (2) et le manchon (18), l'un par rapport à l'autre, autour d'un axe de rotation parallèle à la direction de la ligne centrale longitudinale (C).

2. Dispositif selon la revendication 1, **caractérisé en ce que** le filetage (7) se trouve entre la contre-surface (8a, 8b) et l'extrémité la plus extérieure de l'extrémité distale (4) du dispositif.

3. Dispositif selon la revendication 1, **caractérisé en ce qu'**il comprend deux contre-surfaces : une première contre-surface (8a) disposée de telle sorte que le filetage (7) se trouve entre celle-ci et l'extrémité la plus extérieure de l'extrémité distale (4), et une seconde contre-surface (8b) qui se trouve à l'extrémité la plus extérieure de l'extrémité distale (4).

4. Dispositif selon l'une quelconque des revendications 1 à 3, **caractérisé en ce que** le diamètre extérieur du filetage (7) est invariable substantiellement sur toute sa longueur.

5. Dispositif selon l'une quelconque des revendications précédentes, **caractérisé en ce qu'**un pas du filetage (7) est constant sur toute sa longueur.

6. Dispositif selon la revendication 1, **caractérisé en ce que** le filetage (7) se trouve entre la contre-surface (8a) et l'extrémité la plus extérieure de l'extrémité distale (4) du dispositif, et un foret se trouve à l'extrémité la plus extérieure de l'extrémité distale (4), le foret présentant une coupe à gauche.

7. Dispositif selon l'une quelconque des revendications précédentes, **caractérisé en ce que** la contre surface (8a, 8b) est munie de protubérances se prolongeant dans la direction de la ligne centrale longitudinale (C).

8. Dispositif selon l'une quelconque des revendications précédentes, **caractérisé en ce que** la seconde section filetée (20) présente un filetage à gauche.

9. Dispositif selon l'une quelconque des revendications précédentes, **caractérisé en ce que** la seconde section filetée (20) présente un filetage à droite.
